# EUROPEAN PATENT SPECIFICATION

(11) **EP 4 215 238 B1**
(45) Date of publication and mention of the grant of the patent: **14.02.2024**
(21) Application number: 22152912.6
(22) Date of filing: 24.01.2022
(51) Int. Cl.: A61N 1/32, A61N 1/40

(54) **COSMETIC INSTRUMENT OF HONEYCOMB LATTICE STRUCTURE**
KOSMETISCHES INSTRUMENT MIT WABENFÖRMIGER GITTERSTRUKTUR
INSTRUMENT COSMÉTIQUE À STRUCTURE EN TREILLIS EN FORME DE NID D'ABEILLE

(43) Date of publication of application: 26.07.2023
(73) Proprietor: WuHan Hi-Life Medical Technology Co., Ltd., Wuhan, Hubei 430000 (CN)
(72) Inventor: PAN, Xiaohai, Wuhan, 430000 (CN); XU, Pingjie, Wuhan, 430000 (CN); SHENG, Xiaodong, Wuhan, 430000 (CN); WANG, Zhanghuiyi, Wuhan, 430000 (CN)
(74) Representative: Ipey

(56) References cited:
- CN-U- 210 991 071
- US-A1- 2008 243 039
- US-A1- 2013 312 206

## Description

### Technical Field

The present invention relates to the technical field of therapeutic handles for thermal therapeutic apparatuses, in particular to the technical field of therapeutic handles for thermal therapeutic apparatuses in the medical or beauty industry.

### Background Art

A thermal therapeutic apparatus is a cosmetic instrument which may stretch and firm skin and is high in safety without causing a wound. The principle of the thermal therapeutic apparatus is as follows: by stimulating dermis with a radio-frequency current, synthesis of autologous collagen is promoted, the quality of the skin is improved, wrinkles on the skin can be rapidly removed, and the skin becomes firm and smooth. The action principle of the radio-frequency current is different from the selective photothermolysis of laser light, and the radio-frequency current is converted to heat energy for generating the therapeutic effect after being affected by resistance. A human body is a current conductor. When the radio-frequency current acts on the human body, as the skin and subcutaneous tissues have impedance to electromagnetic waves, water molecules in histiocytes conduct vibration and rotary friction at millions of times per second under the effect of the electromagnetic waves to produce heat, the heat energy acts on target tissues, and thus cells are destroyed, or the cells are gasified, or the tissues are contracted. The dermis of adult skin mainly contains type I collagen, is formed in that three helical peptide chains are bonded by hydrogen bonds, and collagen molecules form stable collagen fibres through covalent crosslinking. After the collagen fibres in the dermis are heated, the size is increased, collagen fibrous septa disappear, and the thermolabile hydrogen bonds are destroyed and curl, so that a part of the collagen fibres are mutually fused, and immediate contraction of the collagen fibres occurs. In addition, tissue thermal damages may further start a wound healing response, that is, activate the activity of fibroblasts and release cytokines and growth factors, wherein the latter promotes formation of the collagen and elastin; and after 12 weeks, original elastic tissues are replaced by the elastin and the collagen regularly arranged in upper dermis, and thus the continuous skin firming effect is generated. At present, the common thermal therapeutic apparatus has the following disadvantages that: firstly, when a traditional micro-current cosmetic instrument is used for therapy, contact sites of the skin with electrodes are pressed by the electrodes, the skin at these sites is unsmoothed in blood circulation, and a cell repair function cannot be fully activated, so that the thermal therapeutic effect is not very ideal; and secondly, after the micro-current cosmetic instrument is used for a long time, the electrodes may be worn and require to be replaced, but for the traditional current cosmetic instrument, a whole handle of the cosmetic instrument requires to be disassembled when the electrodes are replaced, and operation is very troublesome.

CN210991071 U provides a multifunctional cold and hot hammer physiotherapy instrument,which comprises a hammer-shaped shell body, a control circuit board, a massage piece fixedly arranged on the shell body and a first LED lamp assembly arranged on the periphery of the massage piece in a surrounding mode. A semiconductor chilling plate is attached to the back face of the massage piece, and a radiator and a temperature sensor are further arranged on the back face of the semiconductor chilling plate. The other hammer end face of the shell body is further provided with radio frequency electrodes arranged in pairs and a second LED lamp assembly arranged in the gap space of the radio frequency electrodes, and the control circuit board comprises a refrigerating and heating control circuit module, a temperature control circuit module, an LED control circuit module and a radio frequency circuit module. According to the cold and hothammer, one hammer end face of the cold and hot hammer can be used for refrigerating or heating and can also be used for phototherapy at the same time, the other hammer end face of the cold and hot hammer can be used for radio frequency physiotherapy and phototherapy at the same time, and the cold and hot hammer can be used for beautifying or physiotherapy in various modes by combining all functional units of the cold and hot hammer.

US2008/243039A1 discloses an apparatus including a handle capable of manipulation by a human hand, and one or more head portions to mate to various types of treatment attachments, which may be moved over an area of skin and/or body part by a motion generator moving the head portions, and/or by a user manipulating the handle. Various suitable attachments include applicator attachments having abrasive surfaces, oxygenating attachments having pores through which oxygen may travel, brush attachments for cleaning and polishing, thermal attachments for heating and cooling, and light radiating attachments. The motion generator may move the attachments by vibrating, spinning, oscillating, or propagating sonic waves through the head portions. Thus, attachments may be attached and removed from the head portions to treating skin and/or body parts by abrasion, cleaning, polishing, lighting, or oxygenation. Moreover, during treatment abrasive composition, cleaning solution, and/or polishing solution may be applied to the skin and/or body part.

US2013/312206A1 discloses a cosmetic instrument for applying a product to a surface includes a handle and an applicator. At least a portion of the cosmetic instrument may change from a first color to a second color in response to occurrence of a condition, such as exposure to electromagnetic radiation (e.g., ultraviolet light or infrared light) or when a temperature of the cosmetic instrument is within a predetermined temperature range. For example, one or more portions of the handle and/or applicator of the cosmetic instrument may be configured to change color in response to the occurrence of the condition.

### Summary of the Invention

The invention is defined in the appended claims. Aspects, embodiments and examples disclosed herein which do not fall within the scope of the appended claims do not form part of the invention, and are merely provided for illustrative purposes.

The present invention provides a cosmetic instrument with a honeycomb lattice structure and aims at solving the problems that the micro-current cosmetic instrument is not very ideal in thermal therapeutic effect, and electrodes are trouble in replacement operation in the prior art.

The technical solution of the present invention is implemented as follows:
A cosmetic instrument with a honeycomb lattice structure, comprising a handle shell, a lampshade, a lattice head, a circuit board, sleeves and LED lamps; the handle shell is detachably and fixedly connected with the lampshade, the circuit board is mounted in the handle shell, the sleeves are mounted in the handle shell and are electrically connected with the circuit board, the LED lamps are mounted in the handle shell and are electrically connected with the circuit board, characterized in that slot holes are formed in the sleeves respectively; the lattice head comprises barrels and electrodes, electrode tips are arranged at the end parts of the corresponding electrodes, the barrels fixedly penetrate through the lampshade and are conductively inserted into the corresponding slot holes of the sleeves, the electrodes are telescopically conductively inserted into the corresponding barrels through elastic buffer components.

Further, a centre conical table and annularly distributed pyramids are arranged at the end part of each electrode tip, and the pyramids surround the periphery of each centre conical table.

Further, the lattice head further comprises a supporting plate. The supporting plate is detachably and fixedly connected with the lampshade and is provided with positioning sleeves, the barrels penetrate into the corresponding positioning sleeves and are welded and fixed with the corresponding positioning sleeves, the barrels on the supporting plate are distributed in an orthohexagonal lattice, a plate body is arranged in the lampshade, guide holes are formed in the plate body, and the barrels penetrate into the corresponding guide holes.

Further, each elastic buffer component comprises a first pressure spring and a second pressure spring, the electrode arranged in each barrel is provided with a first limiting block, and each barrel is internally provided with a first step face for preventing the first limiting block from escaping from each barrel; each first pressure spring is mounted on the electrode in a sleeved manner, and the two end parts of each first pressure spring are abutted against the first step face and one end surface of the first limiting block respectively; second limiting blocks are arranged on the corresponding cylinders in the barrels, the limiting portions are the end surfaces of the second limiting blocks respectively, each second pressure spring is arranged in the corresponding barrel , and the two end parts of each second pressure spring are abutted against the corresponding limiting portion and the other end surface of a first limiting block respectively.

Further, the cosmetic instrument comprises a vibration mechanism; the vibration mechanism comprises cylinders, a motor, a connecting rod, a seat plate, an eccentric wheel and push rods; the cylinders are movably inserted into the corresponding barrels , and second step faces for preventing the second limiting blocks from escaping from the barrels are arranged in the barrels respectively;, the motor is mounted in the handle shell and is electrically connected with the circuit board, and the eccentric wheel is fixedly connected with an output shaft of the motor; the push plates are fixedly connected with the seat plate, are movably inserted into the corresponding slot holes of the sleeves and are abutted against the end parts of the cylinders, one end of the connecting rod is hinged to the eccentric position of the eccentric wheel, and the other end of the connecting rod is hinged to the seat plate.

Further, a first pin shaft is arranged at the eccentric position of the eccentric wheel, the seat plate is provided with a second pin shaft, connecting sleeves are arranged at the two end parts of the connecting rod respectively, the connecting sleeve at one end part of the connecting rod is mounted on the first pin shaft through a first bearing in the sleeved manner, and the connecting sleeve at the other end part of the connecting rod is mounted on the second pin shaft through a second bearing in the sleeved manner.

Further, the cosmetic instrument further comprises a cable. The cable fixedly penetrates into the handle shell through a cable anti-folding head, line cards are arranged in the handle shell, the cable is clamped on the line cards, one end part of the cable is electrically connected with the circuit board, and an aviation plug is arranged at the other end part of the cable.

Further, the cosmetic instrument further comprises conductive shrapnels. Grooves are formed in the inner side walls of the slot holes of the sleeves respectively, and the conductive shrapnels are arranged in the grooves, are fixedly connected with the sleeves, are electrically connected with the circuit board and make elastic pressure contact with the barrels inserted into the slot holes.

Further, a male rabbet is formed in the handle shell and is provided with a boss, a female rabbet is formed in the lampshade and is provided with a notch, and the lampshade is connected with the male rabbet of the handle shell through the female rabbet in a clamping manner and is buckled on the outer side of the boss through the notch.

By employing the above technical solution, the present invention can have the beneficial effects that:
1. While thermal therapy is conducted by the electrode tips, the electrodes and the electrode tips may be driven by the vibration mechanism to conduct high-frequency reciprocating vibration to exert the massage effect on skin and promote blood circulation, so that the cosmetic result or the physiotherapy effect is significantly improved.
2. When the electrode tips require to be replaced, the supporting plate is disassembled and separated from the lampshade; then the lattice head is pulled towards the outsides of the lampshade, and the barrels are pulled out of the guide holes, through holes and slot holes respectively to complete disassembly of the lattice head; and then the supporting plate of the new lattice head is mounted on the lampshade, the barrels of the new lattice head penetrate into the guide holes, the through holes and the slot holes respectively, and then rapid replacement of the electrode tips may be completed, so that operation is very convenient and rapid, and the time and the labour are saved. In addition, the barrels are fixedly connected with the supporting plate, a plurality of electrodes form a whole body, and all the electrodes are replaced when the lattice head is replaced, so that the influencing effect of mixed use of electrodes to be obsoleted and new electrodes is avoided.
3. The barrels are arranged on the supporting plate in the orthohexagonal lattice. Compared with arrangement in other shapes, with the orthohexagonal arrangement, under an equal area, more electrode tips may be contained, the distribution condition of the electrode tips is that the electrode tips diverge and expand layer by layer from centre to the periphery, and distribution is more reasonable; and meanwhile, the centre conical table is arranged at the end part of the each electrode tip, and the pyramids surround the periphery of each centre conical table, so that distribution of contacts between the electrode tips and the skin are more uniform, and the therapeutic effect may be effectively improved.
4. The LED lamps are arranged in the handle shell. During therapy, light emitted by the LED lamps is emitted from the lampshade, and a certain illumination effect may be exerted, so that a worker may conveniently observe the condition of the skin and accurately position the therapy position on the skin, and the therapeutic effect is further improved

### Brief Description of the Drawings

In order to more clearly describe the technical solutions of the embodiments of the present invention or in the prior art, the accompanying drawings required to describe the embodiments or the prior art are briefly described below. Apparently, the accompanying drawings described below are only some embodiments of the present invention. Those of ordinary skill in the art may further obtain other accompanying drawings based on these accompanying drawings without inventive effort.
Fig. 1 is a structural schematic diagram of the present invention;
Fig. 2 is an enlarged partial schematic diagram at A in Fig. 1;
Fig. 3 is an enlarged partial schematic diagram at B in Fig. 2;
Fig. 4 is a top cross-sectional view of the present invention;
Fig. 5 is an enlarged partial schematic diagram at C in Fig. 2;
Fig. 6 is an enlarged partial schematic diagram at M in Fig. 5;
Fig. 7 is an enlarged partial schematic diagram at N in Fig. 5;
Fig. 8 is an enlarged partial schematic diagram at V in Fig. 5;
Fig. 9 is an enlarged partial schematic diagram at S in Fig. 5; and
Fig. 10 is a Z-Z sectional view in Fig. 5.

In the drawings, reference numerals are used for corresponding components as follows:
1-handle shell, 2-lampshade, 3-circuit board, 4-sleeve, 5-LED lamp, 6-bracket, 7-through hole, 8-guide hole, 9-barrel, 10-electrode tip, 11-electrode, 12-supporting plate, 13-plate body, 14-cylinder, 15-first pressure spring, 16-second pressure spring, 17-first limiting block, 18-first step face, 19-second limiting block, 20-second step face, 21-motor, 22-connecting rod, 23-seat plate, 24-eccentric wheel, 25-push rod, 26-output shaft, 27-first pin shaft, 28-second pin shaft, 29-connecting sleeve, 30-first bearing, 31-second bearing, 32-cable, 33-line card, 34-conductive shrapnel, 35-groove, 36-male rabbet, 37-boss, 38-female rabbet, 39-notch, 40-supporting seat, 41-slot hole, 42-center conical table, 43-pyramid, 44-positioning sleeve, 45-cable anti-folding head, and 46-aviation plug.

### Detailed Description of the Invention

The technical solutions of the present invention are described clearly and completely in combination with the embodiments of the present invention. Apparently, the described embodiments are only part rather than all of the embodiments of the present invention. Based on the embodiments of the present invention, all the other embodiments obtained by those of ordinary skill in the art without inventive effort are within the scope of the present invention.

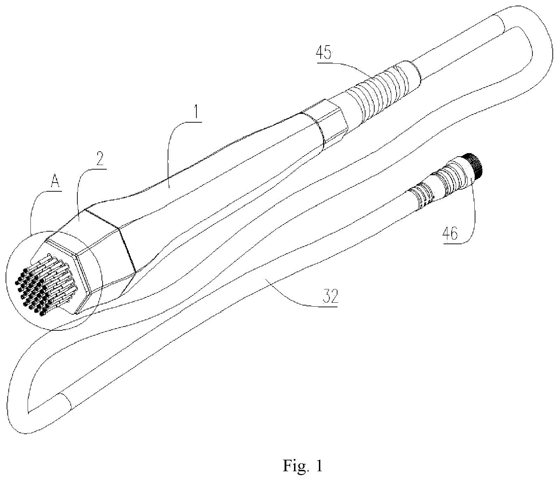

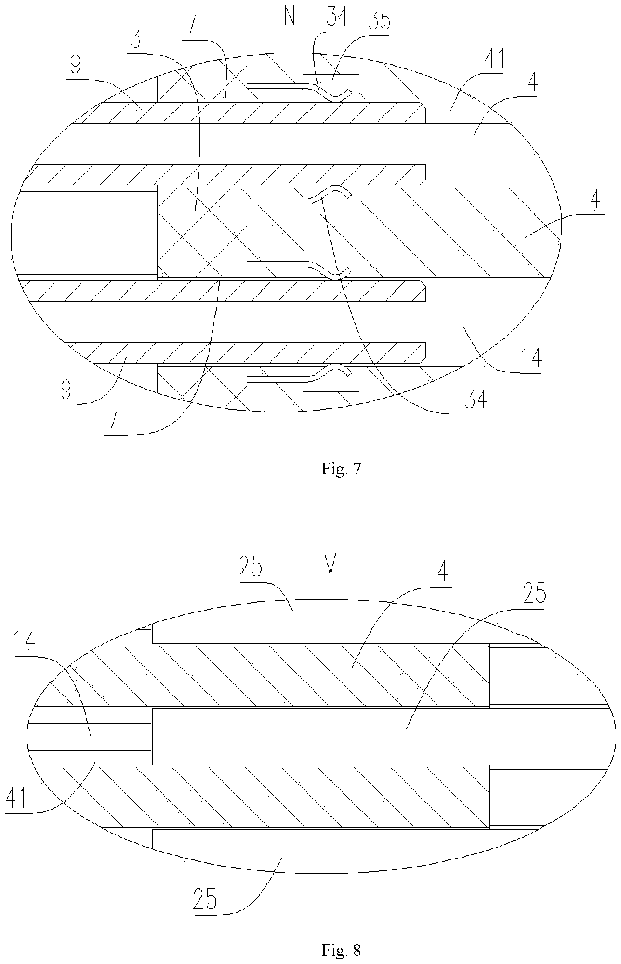

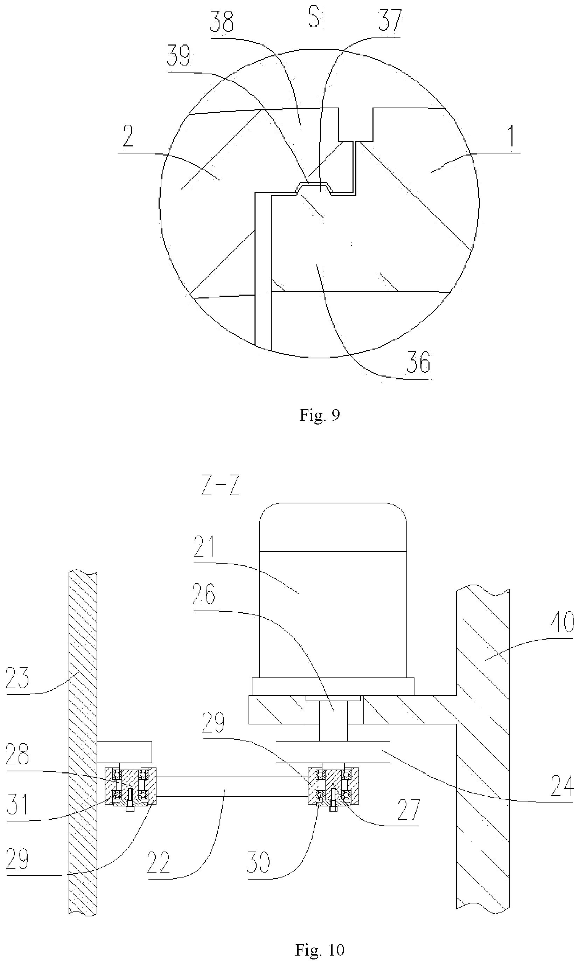

Referring to Fig. 1, Fig. 4 and Fig. 8, disclosed is a cosmetic instrument with a honeycomb lattice structure, comprising a handle shell 1, a lampshade 2, a lattice head, a circuit board 3, sleeves 4, LED lamps 5, a vibration mechanism, a cable 32 and conductive shrapnels 34. The handle shell 1 is detachably and fixedly connected with the lampshade 2; and a specific connection relationship between the handle shell 1 and the lampshade 2 is as follows: referring to Fig. 9, a male rabbet 36 is formed in the handle shell 1 and is provided with a boss 37, a female rabbet 38 is formed in the lampshade 2 and is provided with a notch 39, and the lampshade 2 is connected with the male rabbet 36 of the handle shell 1 through the female rabbet 38 in a clamping manner and is buckled on the outer side of the boss 37 through the notch 39. The handle shell 1 is made of a ABS-acrylonitrile-butadiene-styrene copolymer resin material, is not transparent and is in streamlined appearance transition; and the lampshade 2 is made of a PC-polycarbonate resin material, is semi-transparent and has an appearance in a unique shape.

The circuit board 3 is fixedly mounted in the handle shell 1 through the bracket 6, and the sleeves 4 are mounted in the handle shell 1 and are electrically connected with the circuit board 3. In this embodiment, the sleeves 4 are fixedly mounted on the circuit board 3, slot holes 41 are formed in the sleeves 4 respectively, through holes 7, corresponding to the sleeves 4, are formed in the circuit board 3, the lattice head comprises barrels 9, electrodes 11 and a supporting plate 12, and the barrels 9 fixedly penetrate through the lampshade 2. A specific connection relationship between the barrels 9 and the lampshade 2 is as follows: the supporting plate 12 is detachably and fixedly connected with the lampshade 2, for example, through bolts or buckles, and is provided with positioning sleeves 44, the barrels 9 penetrate into and are welded and fixed with the sleeves 44 respectively, the barrels 9 on the supporting plate 12 are arranged in an orthohexagonal lattice, the plate body 13 is arranged in the supporting plate 12, guide holes 8 are formed in the plate body 13, and the barrels 9 penetrate into the guide holes 8 respectively. The barrels 9 penetrate through the through holes 7 respectively and are conductively inserted into the slot holes 41 of the sleeves 4. A specific electric connection relationship between the barrels 9 and the sleeves 4 is as follows: grooves 35 are formed in the inner side walls of the slot holes 41 of the sleeves 4 respectively, and conductive shrapnels 34 are arranged in the grooves 35 respectively, are fixedly connected with the sleeves 4, are electrically connected with the circuit board 3 and make elastic pressure contact with the barrels 9 inserted into the slot holes 41 respectively.

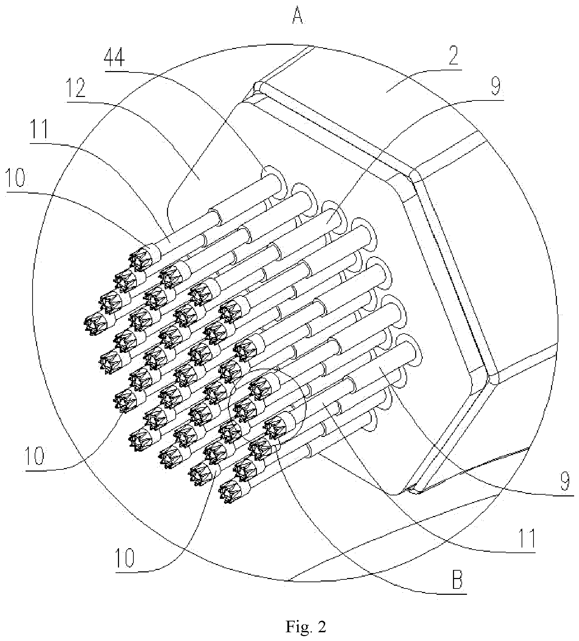

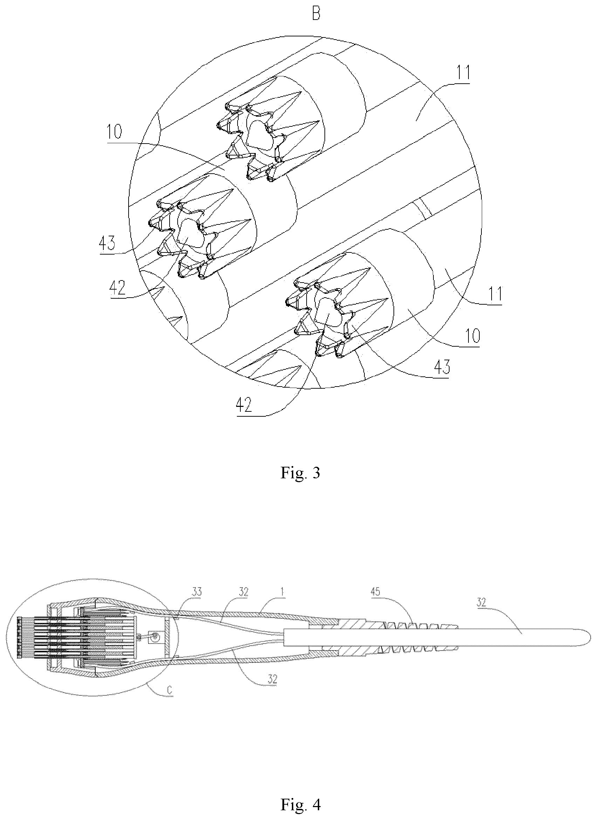

The electrodes 11 are telescopically conductively inserted into the barrels 9 through elastic buffer components respectively, and electrode tips 10 are arranged at the end parts of the electrodes 11 respectively. Referring to Fig. 2 and Fig. 3, a centre conical table 42 and annularly distributed pyramids 43 are arranged at the end part of each electrode tip 10, and the pyramids 43 surround the periphery of each centre conical table 42. A specific structure of each elastic buffer component and a specific connection relationship between the electrodes 11 and the barrels 9 are as follows: each elastic buffer component comprises a first pressure spring 15 and a second pressure spring 16, the electrode 11 arranged in each barrel 9 is provided with a first limiting block 17, each barrel 9 is internally provided with a first step face 18 for preventing the first limiting block 17 from escaping from each barrel 9, each first pressure spring 15 is mounted on the electrode 11 in the sleeved manner, the two end parts of each first pressure spring 15 are abutted against the first step face 18 and one end surface of the first limiting block 17 respectively, a limiting portion is arranged in each barrel 9, each second pressure spring 16 is arranged in the corresponding barrel 9, and the two end parts of each second pressure spring 16 are abutted against the corresponding limiting portion and the other end surface of a first limiting block 17 respectively.

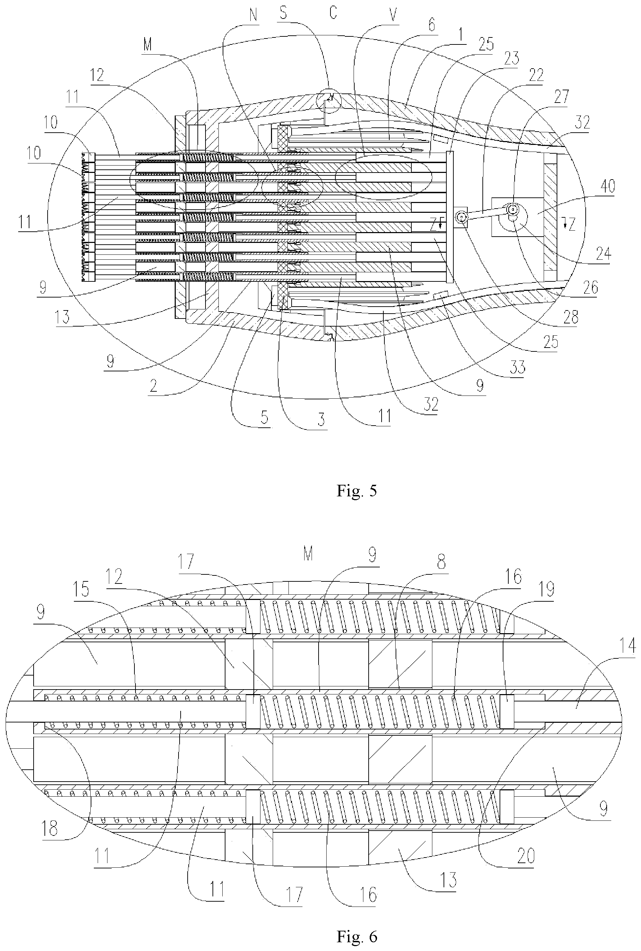

Referring to Fig. 5 and Fig. 10, the vibration mechanism comprises cylinders 14, a motor 21, a connecting rod 22, a seat plate 23, an eccentric wheel 24 and push rods 25, the cylinders 14 are movably inserted into the corresponding barrels 9, second limiting blocks 19 are arranged on the corresponding cylinders 14 in the barrels 9, second step faces 20 for preventing the second limiting blocks 19 from escaping from the barrels 9 are arranged in the barrels 9 respectively, and the limiting portions are the end surfaces of the second limiting blocks 19. The motor 21 is fixedly mounted in the handle shell 1 through the supporting seat 40 and is electrically connected with the circuit board 3, the eccentric wheel 24 is fixedly connected with an output shaft 26 of the motor 21, the push plates 25 are fixedly connected with the seat plate 23, are movably inserted into the corresponding slot holes 41 of the sleeves 4 and are abutted against the end parts of the cylinders 14, one end of the connecting rod 22 is hinged to the eccentric position of the eccentric wheel 24, and the other end of the connecting rod 22 is hinged to the seat plate 23. Specific connection relationships between the connecting rod 22 and the eccentric wheel 24 as well as between the connecting rod 22 and the seat plate 23 are as follows: a first pin shaft 27 is arranged at the eccentric position of the eccentric wheel 24, the seat plate 23 is provided with a second pin shaft 28, connecting sleeves 29 are arranged at the two end parts of the connecting rod 22 respectively, the connecting sleeve 29 at one end part of the connecting rod 22 is mounted on the first pin shaft 27 through a first bearing 30 in the sleeved manner, and the connecting sleeve 29 at the other end part of the connecting rod 22 is mounted on the second pin shaft 28 through a second bearing 31 in the sleeved manner.

The LED lamps 5 are mounted in the handle shell 1. In this embodiment, the LED lamps 5 are fixedly mounted on and are electrically connected with the circuit board 3; and after the LED lamps 5 are lighted on, light is emitted through the lampshade 2. The cable 32 fixedly penetrates into the handle shell 1 through a cable anti-folding head 45, line cards 33 are arranged in the handle shell 1, the cable 32 is clamped on the line cards 33, one end part of the cable 32 is electrically connected with the circuit board 3, and an aviation plug 46 is arranged at the other end part of the cable 32. As being formed by employing a high-quality twisted pair and sleeving the high-quality twisted pair with a cotton yarn+PET tightly woven net line protective sleeve, the cable 32 resists high temperature, corrosion and bending, is long in service life and has very good insulating performance. By employing a waterproof aviation plug SP13, the aviation plug 46 is strong in sealing performance and may ensure that oil stains and water stains cannot enter the cosmetic instrument along the cable to cause a short circuit at a connector in the use process.

When the cosmetic instrument with the honeycomb lattice structure of the present invention is used, through electric connection between the aviation plug 46 and a host machine of the thermal therapeutic apparatus, a worker holds the handle shell 1 by a hand to enable electrodes 10 to make contact with skin of a human body; the circuit board 3 is controlled with the host machine of the thermal therapeutic apparatus to generate particular voltage and current, and a safe and reliable high-frequency electric field is formed; and the current is conducted to the electrode tips 10 through the sleeves 4, the barrels 9 and the electrodes 11 and acts on the human skin through the electrode tips 10 to generate the warm effect, so that thermal therapy and the cosmetic result or the physiotherapy effect are achieved. While thermal therapy is conducted, the electrodes 11 and the electrode tips 10 may be driven by the vibration mechanism to conduct reciprocating vibration to exert the massage effect on the skin and promote blood circulation, so that the cosmetic result or the physiotherapy effect is significantly improved. The principle that the vibration mechanism drives the electrodes 11 and the electrode tips 10 to conduct reciprocating vibration is as follows: the eccentric wheel 24 is driven by the motor 21 to rotate, the seat plate 23 and the push rods 25 are driven by the connecting rod 22 to conduct reciprocating vibration along the slot holes 41 of the sleeves 4, the cylinders 14 are rapidly pushed back and forth by the push rods 25, then reciprocating motion is transferred to the electrodes 11 through the second pressure springs 16, and then the electrodes 11 and the electrode tips 10 are driven to conduct high-frequency rapid vibration, so that a massage function of the electrode tips 10 is achieved. It should be noted that, in order to ensure that the cylinders 14 can effectively transfer the reciprocating motion to the electrodes 11 through the second pressure springs 16, when stiffness factors of the first pressure springs 15 and the second pressure springs 16 are selected, the stiffness factor of the second pressure springs 16 should be larger than that of the first pressure springs 15. After the cosmetic instrument with the honeycomb lattice structure is used for a long time, the electrode tips 10 of the lattice head may be worn to a certain degree; and when the electrode tips 10 require to be replaced after being worn to a certain degree, the supporting plate 12 and the lampshade 2 may be disassembled and separated, then the lattice head is pulled towards the outsides of the lampshade 2, the barrels 9 are pulled out of the guide holes 8, the through holes 7 and the slot holes 41 respectively to complete disassembly of the lattice head, then the supporting plate 12 of a new lattice head is mounted on the lampshade 2 again, the barrels 9 of the new lattice head penetrate into the guide holes 8, the through holes 7 and the slot holes 41 respectively, and then the electrode tips 10 are rapidly replaced.

## Claims

1. A cosmetic instrument with a honeycomb lattice structure, comprising a handle shell (1), a lampshade (2), a lattice head, a circuit board (3), sleeves (4) and LED lamps (5); the handle shell (1) is detachably and fixedly connected with the lampshade (2), the circuit board (3) is mounted in the handle shell (1), the sleeves (4) are mounted in the handle shell (1) and are electrically connected with the circuit board (3), the LED lamps (5) are mounted in the handle shell (1) and are electrically connected with the circuit board (3), **characterized in that** slot holes (41) are formed in the sleeves (4) respectively; the lattice head comprises barrels (9) and electrodes (11), electrode tips (10) are arranged at the end parts of the corresponding electrodes (11), the barrels (9) fixedly penetrate through the lampshade (2) and are conductively inserted into the corresponding slot holes (41) of the sleeves (4), the electrodes (11) are telescopically conductively inserted into the corresponding barrels (9) through elastic buffer components.

2. The cosmetic instrument with the honeycomb lattice structure according to claim 1, **characterized in that** a center conical table (42) and annularly distributed pyramids (43) are arranged at the end part of each electrode tip (10), and the pyramids (43) surround the periphery of each center conical table (42).

3. The cosmetic instrument with the honeycomb lattice structure according to claim 1, **characterized in that** the lattice head further comprises a supporting plate (12); the supporting plate (12) is detachably and fixedly connected with the lampshade (2) and is provided with positioning sleeves (44), the barrels (9) penetrate into the corresponding positioning sleeves (44) and are welded and fixed with the corresponding positioning sleeves (44), the barrels (9) on the supporting plate (12) are distributed in an orthohexagonal lattice, a plate body (13) is arranged in the lampshade (2), guide holes (8) are formed in the plate body (13), and the barrels (9) penetrate into the corresponding guide holes (8).

4. The cosmetic instrument with the honeycomb lattice structure according to claim 1, **characterized in that** each elastic buffer component comprises a first pressure spring (15) and a second pressure spring (16), the electrode (11) arranged in each barrel (9) is provided with a first limiting block (17), and each barrel (9) is internally provided with a first step face (18) for preventing the first limiting block (17) from escaping from each barrel (9); each first pressure spring (15) is mounted on the electrode (11) in a sleeved manner, and the two end parts of each first pressure spring (15) are abutted against the first step face (18) and one end surface of the first limiting block (17) respectively; second limiting blocks (19) are arranged on the corresponding cylinders (14) in the barrels (9), the limiting portions are the end surfaces of the second limiting blocks (19) respectively, each second pressure spring (16) is arranged in the corresponding barrel (9), and the two end parts of each second pressure spring (16) are abutted against the corresponding limiting portion and the other end surface of a first limiting block (17) respectively.

5. The cosmetic instrument with the honeycomb lattice structure according to claim 4, **characterized in that** the cosmetic instrument further comprises a vibration mechanism; the vibration mechanism comprises cylinders (14), a motor (21), a connecting rod (22), a seat plate (23), an eccentric wheel (24) and push rods (25); the cylinders (14) are movably inserted into the corresponding barrels (9), and second step faces (20) for preventing the second limiting blocks (19) from escaping from the barrels (9) are arranged in the barrels (9) respectively;, the motor (21) is mounted in the handle shell (1) and is electrically connected with the circuit board (3), and the eccentric wheel (24) is fixedly connected with an output shaft (26) of the motor (21); the push plates (25) are fixedly connected with the seat plate (23), are movably inserted into the corresponding slot holes (41) of the sleeves (4) and are abutted against the end parts of the cylinders (14), one end of the connecting rod (22) is hinged to the eccentric position of the eccentric wheel (24), and the other end of the connecting rod (22) is hinged to the seat plate (23).

6. The cosmetic instrument with the honeycomb lattice structure according to claim 5, **characterized in that** a first pin shaft (27) is arranged at the eccentric position of the eccentric wheel (24), the seat plate (23) is provided with a second pin shaft (28), connecting sleeves (29) are arranged at the two end parts of the connecting rod (22) respectively, the connecting sleeve (29) at one end part of the connecting rod (22) is mounted on the first pin shaft (27) through a first bearing (30) in the sleeved manner, and the connecting sleeve (29) at the other end part of the connecting rod (22) is mounted on the second pin shaft (28) through a second bearing (31) in the sleeved manner.

7. The cosmetic instrument with the honeycomb lattice structure according to claim 1, **characterized in that** the cosmetic instrument further comprises a cable (32); the cable (32) fixedly penetrates into the handle shell (1) through a cable anti-folding head (45), line cards (33) are arranged in the handle shell (1), the cable (32) is clamped on the line cards (33), one end part of the cable (32) is electrically connected with the circuit board (3), and an aviation plug (46) is arranged at the other end part of the cable (32).

8. The cosmetic instrument with the honeycomb lattice structure according to claim 1, **characterized in that** the cosmetic instrument further comprises conductive shrapnels (34); grooves (35) are formed in the inner side walls of the slot holes (41) of the sleeves (4) respectively; and the conductive shrapnels (34) are arranged in the grooves (35), are fixedly connected with the sleeves (4), are electrically connected with the circuit board (3) and make elastic pressure contact with the barrels (9) inserted into the slot holes (41).

9. The cosmetic instrument with the honeycomb lattice structure according to claim 1, **characterized in that** a male rabbet (36) is formed in the handle shell (1) and is provided with a boss (37); a female rabbet (38) is formed in the lampshade (2) and is provided with a notch (39); and the lampshade (2) is connected with the male rabbet (36) of the handle shell (1) through the female rabbet (38) in a clamping manner and is buckled on the outer side of the boss (37) through the notch (39).

## Patentansprüche

1. Kosmetisches Instrument mit wabenförmiger Gitterstruktur, umfassend eine Griffhülle (1), einen Lampenschirm (2), einen Gitterkopf, eine Leiterplatte (3), Buchsen (4) und LED-Lampen (5); wobei die Griffhülle (1) lösbar und fest mit dem Lampenschirm (2) verbunden ist, die Leiterplatte (3) in der Griffhülle (1) montiert ist, die Buchsen (4) in der Griffhülle (1) montiert sind und elektrisch mit der Leiterplatte (3) verbunden sind, die LED-Lampen (5) in der Griffhülle (1) montiert sind und elektrisch mit der Leiterplatte (3) verbunden sind, **dadurch gekennzeichnet, dass** in den Buchsen (4) jeweils Schlitzlöcher (41) gebildet sind, der Gitterkopf Hülsen (9) und Elektroden (11) umfasst, Elektrodenspitzen (10) an den Endteilen der entsprechenden Elektroden (11) angeordnet sind, die Hülsen (9) den Lampenschirm (2) fest durchdringen und leitend in die entsprechenden Schlitzlöcher (41) der Buchsen (4) eingefügt sind und die Elektroden (11) durch elastische Pufferkomponenten teleskopartig leitend in die entsprechenden Hülsen (9) eingefügt sind.

2. Kosmetisches Instrument mit der wabenförmigen Gitterstruktur nach Anspruch 1, **dadurch gekennzeichnet, dass** eine zentrale Kegelplatte (42) und ringförmig verteilte Pyramiden (43) am Endteil jeder Elektrodenspitze (10) angeordnet sind und die Pyramiden (43) den Umfang jeder zentralen Kegelplatte (42) umgeben.

3. Kosmetisches Instrument mit der wabenförmigen Gitterstruktur nach Anspruch 1, **dadurch gekennzeichnet, dass** der Gitterkopf ferner eine Stützplatte (12) umfasst; wobei die Stützplatte (12) lösbar und fest mit dem Lampenschirm (2) verbunden ist und mit Positionierungsbuchsen (44) versehen ist, die Hülsen (9) in die entsprechenden Positionierungsbuchsen (44) eindringen und mit den entsprechenden Positionierungsbuchsen (44) verschweißt und an diesen befestigt sind, die Hülsen (9) auf der Stützplatte (12) in einem orthohexagonalen Gitter verteilt sind, ein Plattenkörper (13) in dem Lampenschirm (2) angeordnet ist, Führungslöcher (8) in dem Plattenkörper (13) gebildet sind und die Hülsen (9) in die entsprechenden Führungslöcher (8) eindringen.

4. Kosmetisches Instrument mit der wabenförmigen Gitterstruktur nach Anspruch 1, **dadurch gekennzeichnet, dass** jede elastische Pufferkomponente eine erste Druckfeder (15) und eine zweite Druckfeder (16) umfasst, die in jeder Hülse (9) angeordnete Elektrode (11) mit einem ersten Begrenzungsblock (17) versehen ist und jede Hülse (9) innen mit einer ersten Stufenfläche (18) versehen ist, um zu verhindern, dass der erste Begrenzungsblock (17) aus jeder Hülse (9) austritt; wobei jede erste Druckfeder (15) auf hülsenartige Weise an der Elektrode (11) montiert ist und die beiden Endteile jeder ersten Druckfeder (15) an der ersten Stufenfläche (18) bzw. an einer Endfläche des ersten Begrenzungsblocks (17) anliegen; wobei zweite Begrenzungsblöcke (19) an den entsprechenden Zylindern (14) in den Hülsen (9) angeordnet sind, die Begrenzungsabschnitte jeweils die Endflächen der zweiten Begrenzungsblöcke (19) sind, jede zweite Druckfeder (16) in der entsprechenden Hülse (9) angeordnet ist und die beiden Endteile jeder zweiten Druckfeder (16) an dem entsprechenden Begrenzungsabschnitt bzw. an der anderen Endfläche eines ersten Begrenzungsblocks (17) anliegen.

5. Kosmetisches Instrument mit der wabenförmigen Gitterstruktur nach Anspruch 4, **dadurch gekennzeichnet, dass** das kosmetische Instrument ferner einen Vibrationsmechanismus umfasst; wobei der Vibrationsmechanismus Zylinder (14), einen Motor (21), eine Verbindungsstange (22), eine Sitzplatte (23), ein Exzenterrad (24) und Schubstangen (25) umfasst; wobei die Zylinder (14) beweglich in die entsprechenden Hülsen (9) eingefügt sind und in den Hülsen (9) jeweils zweite Stufenflächen (20) angeordnet sind, um zu verhindern, dass die zweiten Begrenzungsblöcke (19) aus den Hülsen (9) austreten; wobei der Motor (21) in der Griffhülle (1) montiert ist und elektrisch mit der Leiterplatte (3) verbunden ist und das Exzenterrad (24) fest mit einer Ausgangswelle (26) des Motors (21) verbunden ist; wobei die Schubplatten (25) fest mit der Sitzplatte (23) verbunden sind, beweglich in die entsprechenden Schlitzlöcher (41) der Buchsen (4) eingefügt sind und an den Endteilen der Zylinder (14) anliegen, ein Ende der Verbindungsstange (22) an die exzentrische Position des Exzenterrads (24) angelenkt ist und das andere Ende der Verbindungsstange (22) an die Sitzplatte (23) angelenkt ist.

6. Kosmetisches Instrument mit der wabenförmigen Gitterstruktur nach Anspruch 5, **dadurch gekennzeichnet, dass** eine erste Zapfenwelle (27) an der exzentrischen Position des Exzenterrads (24) angeordnet ist, die Sitzplatte (23) mit einer zweiten Zapfenwelle (28) versehen ist, Verbindungsbuchsen (29) jeweils an den beiden Endteilen der Verbindungsstange (22) angeordnet sind, die Verbindungsbuchse (29) an einem Endteil der Verbindungsstange (22) durch ein erstes Lager (30) auf hülsenartige Weise an der ersten Zapfenwelle (27) montiert ist und die Verbindungsbuchse (29) an dem anderen Endteil der Verbindungsstange (22) durch ein zweites Lager (31) auf hülsenartige Weise an der zweiten Zapfenwelle (28) montiert ist.

7. Kosmetisches Instrument mit der wabenförmigen Gitterstruktur nach Anspruch 1, **dadurch gekennzeichnet, dass** das kosmetische Instrument ferner ein Kabel (32) umfasst; wobei das Kabel (32) durch einen Kabelknickschutzkopf (45) fest in die Griffhülle (1) eindringt, Leitungskarten (33) in der Griffhülle (1) angeordnet sind, das Kabel (32) an die Leitungskarten (33) geklemmt ist, ein Endteil des Kabels (32) elektrisch mit der Leiterplatte (3) verbunden ist und ein Luftfahrtstecker (46) am anderen Endteil des Kabels (32) angeordnet ist.

8. Kosmetisches Instrument mit der wabenförmigen Gitterstruktur nach Anspruch 1, **dadurch gekennzeichnet, dass** das kosmetische Instrument ferner leitende Splitter (34) umfasst; wobei Nuten (35) jeweils in den Innenseitenwänden der Schlitzlöcher (41) der Buchsen (4) gebildet sind und die leitenden Splitter (34) in den Nuten (35) angeordnet sind, fest mit den Buchsen (4) verbunden sind, elektrisch mit der Leiterplatte (3) verbunden sind und in elastischem Druckkontakt mit den Hülsen (9) stehen, die in die Schlitzlöcher (41) eingefügt sind.

9. Kosmetisches Instrument mit der wabenförmigen Gitterstruktur nach Anspruch 1, **dadurch gekennzeichnet, dass** ein männlicher Falz (36) in der Griffhülle (1) ausgebildet ist und mit einem Vorsprung (37) versehen ist, ein weiblicher Falz (38) in dem Lampenschirm (2) ausgebildet ist und mit einer Kerbe (39) versehen ist und der Lampenschirm (2) durch den weiblichen Falz (38) auf klemmende Weise mit dem männlichen Falz (36) der Griffhülle (1) verbunden ist und durch die Kerbe (39) an die Außenseite des Vorsprungs (37) angelegt ist.

## Revendications

1. Instrument cosmétique à structure en treillis en nid-d'abeilles, comprenant une coque de poignée (1), un abat-jour (2), une tête en treillis, une carte de circuit imprimé (3), des manchons (4) et des lampes DEL (5) ; la coque de poignée (1) est raccordée de manière amovible et fixe à l'abat-jour (2), la carte de circuit imprimé (3) est montée dans la coque de poignée (1), les manchons (4) sont montés dans la coque de poignée (1) et sont raccordés électriquement à la carte de circuit imprimé (3), les lampes DEL (5) sont montées dans la coque de poignée (1) et sont raccordées électriquement à la carte de circuit imprimé (3), **caractérisé en ce que** des trous oblongs (41) sont formés dans les manchons (4), respectivement ; la tête en treillis comprend des barillets (9) et des électrodes (11), des pointes d'électrodes (10) sont disposées au niveau des parties d'extrémité des électrodes (11) correspondantes, les barillets (9) pénètrent de manière fixe à travers l'abat-jour (2) et sont insérés de manière conductrice dans les trous oblongs (41) correspondants des manchons (4), les électrodes (11) sont insérées télescopiquement de manière conductrice dans les barillets (9) correspondants par l'intermédiaire de composants tampons élastiques.

2. Instrument cosmétique à structure en treillis en nid-d'abeilles selon la revendication 1, **caractérisé en ce qu'**une table conique centrale (42) et des pyramides (43) réparties annulairement sont disposées sur la partie d'extrémité de chaque pointe d'électrode (10), et les pyramides (43) entourent la périphérie de chaque table conique centrale (42).

3. Instrument cosmétique à structure en treillis en nid-d'abeilles selon la revendication 1, **caractérisé en ce que** la tête en treillis comprend en outre une plaque de support (12) ; la plaque de support (12) est raccordée de manière amovible et fixe à l'abat-jour (2) et est pourvue de manchons de positionnement (44), les barillets (9) pénètrent dans les manchons de positionnement (44) correspondants et sont soudés et fixés avec les manchons de positionnement (44) correspondants, les barillets (9) sur la plaque de support (12) sont répartis en un réseau orthohexagonal, un corps de plaque (13) est disposé dans l'abat-jour (2), des trous de guidage (8) sont formés dans le corps de plaque (13), et les barillets (9) pénètrent dans les trous de guidage (8) correspondants.

4. Instrument cosmétique à structure en treillis en nid-d'abeilles selon la revendication 1, **caractérisé en ce que** chaque composant tampon élastique comprend un premier ressort de pression (15) et un second ressort de pression (16), l'électrode (11) disposée dans chaque barillet (9) est pourvue d'un premier bloc de limitation (17), et chaque barillet (9) est pourvu intérieurement d'une première face à gradins (18) pour empêcher le premier bloc de limitation (17) de s'échapper de chaque barillet (9) ; chaque premier ressort de pression (15) est monté sur l'électrode (11) de manière manchonnée, et les deux parties d'extrémité de chaque premier ressort de pression (15) sont en butée contre la première face à gradins (18) et une surface d'extrémité du premier bloc de limitation (17) respectivement ; des seconds blocs de limitation (19) sont disposés sur les cylindres (14) correspondants dans les barillets (9), les portions de limitation sont les surfaces d'extrémité des seconds blocs de limitation (19) respectivement, chaque second ressort de pression (16) est disposé dans le barillet (9) correspondant, et les deux parties d'extrémité de chaque second ressort de pression (16) sont en butée contre la portion de limitation correspondante et l'autre surface d'extrémité d'un premier bloc de limitation (17) respectivement.

5. Instrument cosmétique à structure en treillis en nid-d'abeilles selon la revendication 4, **caractérisé en ce que** l'instrument cosmétique comprend en outre un mécanisme de vibration ; le mécanisme de vibration comprend des cylindres (14), un moteur (21), une bielle (22), une plaque de siège (23), une roue excentrique (24) et des biellettes de poussée (25) ; les cylindres (14) sont insérés de manière mobile dans les barillets (9) correspondants, et des secondes faces à gradins (20) pour empêcher les seconds blocs de limitation (19) de s'échapper des barillets (9) sont disposées dans les barillets (9) respectivement ; le moteur (21) est monté dans la coque de poignée (1) et est raccordé électriquement à la carte de circuit imprimé (3), et la roue excentrique (24) est raccordée de manière fixe à un arbre de sortie (26) du moteur (21) ; les plaques de poussée (25) sont raccordées de manière fixe à la plaque de siège (23), sont insérées de manière mobile dans les trous oblongs (41) correspondants des manchons (4) et sont en butée contre les parties d'extrémité des cylindres (14), une extrémité de la bielle (22) est articulée sur la position excentrique de la roue excentrique (24), et l'autre extrémité de la bielle (22) est articulée sur la plaque de siège (23) .

6. Instrument cosmétique à structure en treillis en nid-d'abeilles selon la revendication 5, **caractérisé en ce qu'**un premier arbre à broches (27) est disposé à la position excentrique de la roue excentrique (24), la plaque de siège (23) est pourvue d'un second arbre à broches (28), des manchons de raccordement (29) sont disposés au niveau des deux parties d'extrémité de la bielle (22) respectivement, le manchon de raccordement (29) au niveau d'une partie d'extrémité de la bielle (22) est monté sur le premier arbre à broches (27) par l'intermédiaire d'un premier palier (30) de manière manchonnée, et le manchon de raccordement (29) au niveau de l'autre partie d'extrémité de la bielle (22) est monté sur le second arbre à broches (28) par l'intermédiaire d'un second palier (31) de manière manchonnée.

7. Instrument cosmétique à structure en treillis en nid-d'abeilles selon la revendication 1, **caractérisé en ce que** l'instrument cosmétique comprend en outre un câble (32) ; le câble (32) pénètre de manière fixe dans la coque de poignée (1) à travers une tête anti-pliage de câble (45), des cartes de ligne (33) sont disposées dans la coque de poignée (1), le câble (32) est serré sur les cartes de ligne (33), une partie d'extrémité du câble (32) est raccordée électriquement à la carte de circuit imprimé (3), et une fiche aviation (46) est disposée au niveau de l'autre partie d'extrémité du câble (32).

8. Instrument cosmétique à structure en treillis en nid-d'abeilles selon la revendication 1, **caractérisé en ce que** l'instrument cosmétique comprend en outre des shrapnels conducteurs (34) ; des rainures (35) sont formées dans les parois latérales internes des trous oblongs (41) des manchons (4) respectivement ; et les shrapnels conducteurs (34) sont disposés dans les rainures (35), sont raccordés de manière fixe aux manchons (4), sont raccordés électriquement à la carte de circuit imprimé (3) et établissent un contact de pression élastique avec les barillets (9) insérés dans les trous oblongs (41).

9. Instrument cosmétique à structure en treillis en nid-d'abeilles selon la revendication 1, **caractérisé en ce qu'**une feuillure mâle (36) est formée dans la coque de poignée (1) et est pourvue d'un bossage (37) ; une feuillure femelle (38) est formée dans l'abat-jour (2) et est pourvue d'une encoche (39) ; et l'abat-jour (2) est raccordé à la feuillure mâle (36) de la coque de poignée (1) par l'intermédiaire de la feuillure femelle (38) de manière à être serré et est bouclé sur le côté externe du bossage (37) par l'intermédiaire de l'encoche (39).
